(19) Europäisches Patentamt
European Patent Office
Office européen des brevets

(11) EP 0 675 188 B1

# (12) EUROPÄISCHE PATENTSCHRIFT

(45) Veröffentlichungstag und Bekanntmachung des Hinweises auf die Patenterteilung:
**28.04.1999 Patentblatt 1999/17**

(51) Int Cl.[6]: **C09K 19/58**, C07D 317/32

(21) Anmeldenummer: **95104062.5**

(22) Anmeldetag: **20.03.1995**

(54) **Optisch aktive Tolan-Derivate**

Optically active tolane derivative

Dérivé de tolane optiquement actif

(84) Benannte Vertragsstaaten:
**CH DE FR GB IT LI NL**

(30) Priorität: **30.03.1994 CH 952/94**

(43) Veröffentlichungstag der Anmeldung:
**04.10.1995 Patentblatt 1995/40**

(73) Patentinhaber: **Rolic AG**
**6301 Zug (CH)**

(72) Erfinder:
- **Buchecker, Richard**
  **CH-8008 Zürich (CH)**
- **Marck, Guy**
  **F-68460 Lutterbach (FR)**
- **Schadt, Martin**
  **CH-4411 Seltisberg (CH)**

(74) Vertreter: **England, Christopher David et al**
**BTG International Ltd.,**
**10,Fleet Place,**
**Limeburner Lane**
**London EC4M 7SB (GB)**

(56) Entgegenhaltungen:
**EP-A- 0 441 213** **EP-A- 0 534 258**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue chirale Dotierstoffe für Flüssigkristalle sowie flüssigkristalline Gemische, die solche Dotierstoffe enthalten, und deren Verwendung für optische und elektro-optische Vorrichtungen.

Flüssigkristallmaterialien für cholesterische Filter enthalten ausser nematischen Flüssigkristallen häufig einen oder mehrere optisch aktive Zusatzstoffe zur Induzierung einer chiralen Struktur. Beispielsweise wird zur Verwendung solcher Flüssigkristalle in optischen Filtern ein nematischer Flüssigkristall mit optisch aktivem Zusatzstoff so dotiert, dass einerseits eine bestimmte Drehrichtung der Helix entsteht und sich andererseits die gewünschte Helixganghöhe einstellt. Ausser den chiralen Eigenschaften von Dotierstoffen sind auch bestimmte dielektrische und optische Eigenschaften für den optimalen Einsatz in cholesterischen Filtern wünschenwert. Um eine möglichst grosse Bandbreite der selektiven Filter-Reflektion zu erhalten, werden beispielsweise chirale Dotierstoffe mit grosser optischer Anisotropie benötigt. Ferner lassen sich mit negativ dielektrisch anisotropen Dotierstoffen cholesterische Filter bauen, die sich im elektrischen Feld planar umd damit disinklinationsfrei orientieren lassen. Dadurch kann eine hohe optische Qualität der Filter realisiert werden, [M. Schadt, Liquid Crystals 14, 73 (1993)].

Cholesterische Flüssigkristalle reflektieren selektiv Licht in einem Wellenlängenbereich, für den die Wellenlänge etwa gleich der Helixganghöhe ist. Die spektrale Breite dieses Reflexionslichtes kann durch geeignete Wahl des Flüssigkristalles variiert werden. Das reflektierte Licht ist vollständig zirkular polarisiert. Die Drehrichtung des reflektierten Lichts hängt vom Drehsinn der cholesterischen Helixstruktur ab. Das entgegengesetzt zirkular polarisierte Licht wird ungeschwächt transmittiert. Diese Eigenschaften können zur Herstellung von optischen Filtern, Polarisatoren, Analysatoren etc. ausgenutzt werden.

Cholesterische Flüssigkristalle für obige Anwendungen können vorzugsweise aus einem nematischen oder cholesterischen Basismaterial und einem oder mehreren chiralen Dotierstoffen bestehen, was eine einfache Einstellung der gewünschten Helixganghöhe erlaubt.

Um cholesterische Gemische mit einer Ganghöhe im Bereich der Wellenlänge des sichtbaren Lichts zu erreichen, sollten die chiralen Dotierstoffe ein möglichst hohes Verdrillungsvermögen aufweisen und in üblichen Flüssigkristallmaterialien gut löslich sein. Weiterhin sollten die chiralen Dotierstoffe eine ausreichende Stabilität besitzen, mit dem Mesophasetyp des Flüssigkristallmaterials gut kompatibel sein und den Mesophasenbereich nicht zu stark einschränken. Solche Eigenschaften wären auch für chirale Dotierstoffe zur Erzielung der eingangs genannten hochverdrillten nematischen Strukturen erwünscht, damit ihr Anteil so niedrig gehalten werden kann, dass die übrigen Eigenschaften des Flüssigkristall-Materials nur unwesentlich beeinflusst werden. Dotierstoffe, welche obige Zwecke zum Teil erfüllen, sind bereits bekannt,so sind zum Beispiel in EP-A-0 441 213 optisch aktive Dioxolan Derivate als Dotierstoffe für Flüssigkristallmischungen beschrieben.

Die Erfindung betrifft optisch aktive Verbindungen der allgemeinen Formel

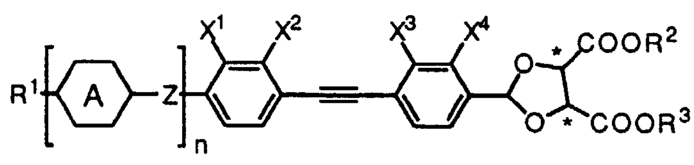

I

worin

| | |
|---|---|
| Ring A | unsubstituiertes oder einfach oder mehrfach substituiertes 1,4-Phenylen, in welchem gegebenenfalls eine oder zwei CH-Gruppen durch Stickstoff ersetzt sein können, oder trans-1,4-Cyclohexylen bedeutet; |
| Z | eine einfache Kovalenzbindung, $-CH_2CH_2-$, $-CH_2O-$ oder $-OCH_2-$ bedeutet; |
| $X^1$, $X^2$, $X^3$, $X^4$ | unabhängig voneinander Wasserstoff oder Fluor darstellen; |
| $R^1$ | Wasserstoff, unsubstituiertes, einfach oder mehrfach mit Fluor oder Chlor substituiertes Alkyl oder Alkenyl mit 1 bzw. 2 bis 12 Kohlenstoffatomen, in welchen auch eine oder zwei nicht benachbarte $CH_2$-Gruppen durch -O-ersetzt sein können, oder eine Gruppe der Formel |

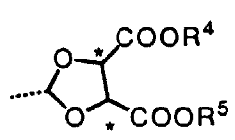

(a)

| | |
|---|---|
| | oder - falls $R^1$ an einen aromatischen Ring gebunden ist - auch Fluor, Chlor, Cyano, $-CF_3$, $-OCHF_2$ oder $-OCF_3$ bedeutet; |
| $R^2$, $R^3$, $R^4$, $R^5$ | Alkyl oder Alkenyl mit 1 bzw. 2 bis 8 Kohlenstoffatomen bedeuten; |
| n | 0 oder 1 bedeutet; und |
| * | ein Chiralitätszentrum markiert. |

Die Verbindungen der Formel I besitzen somit mindestens einen optisch aktiven Dioxolan-Ring. Der in Formel I dargestellte Dioxolan-Ring besitzt in den Positionen 4 und 5 (d.h. benachbart zu den Resten $-COOR^2$ und $-COOR^3$) je ein chirales Kohlenstoffatom. Es ist dem Fachmann klar, dass zur Erzielung optischer Aktivität das Kohlenstoffatom in Position 4 des Dioxolan-Ringes ganz oder überwiegend in R- oder S-Form vorliegen muss und, falls $R^2$ und $R^3$ identisch sind, das Kohlenstoffatom in Position 5 des Dioxolan-Ringes ganz oder überwiegend die gleiche Konfiguration aufweisen sollte wie das Kohlenstoffatom in Position 4 oder grundsätzlich auch ein R/S-Verhältnis von 50:50 aufweisen kann. Um ein möglichst hohes Verdrillungsvermögen zu erzielen, sollte das Kohlenstoffatom in Position 4 des Dioxolan-Ringes vorzugsweise in optisch möglichst reiner Form in der R- oder S-Konfiguration vorliegen und das Kohlenstoffatom in Position 5 des Dioxolan-Ringes vorzugsweise in optisch möglichst reiner Form in jener Konfiguration vorliegen, die das Verdrillungsvermögen verstärkt. Bevorzugte optische Isomere mit hohen Verdrillungsvermögen sind die (4R, 5R)-Isomeren und die (4S,5S)-Isomeren der Verbindungen der Formel I.

Die Verbindungen der Formel I sind sehr gut löslich in üblichen Flüssigkristallmaterialien und ermöglichen eine sehr hohe Verdrillung der Flüssigkristallstruktur. Im Unterschied zu bekannten Materialien mit hohen Verdrillungsvermögen werden zudem die Klärpunkte von Flüssigkristallen bei Zusatz von Verbindungen der Formel I in der Regel nicht oder nur unwesentlich gesenkt. Viele der erfindungsgemässen Verbindungen besitzen sogar selbst flüssigkristalline Eigenschaften. Die Verbindungen der Formel I sind leicht herstellbar, relativ niederviskos und ausreichend stabil gegen elektrische und magnetische Felder. Sie erfüllen daher in optimaler Weise die oben genannten Anforderungen.

Die Eigenschaften der Verbindungen der Formel I können je nach Zahl und Bedeutung des Ringes A und der Substituenten $R^1$, $R^2$, $R^3$, $X^1$, $X^2$, $X^3$ und $X^4$ variiert werden. Beispielsweise führt ein zusätzlicher aromatischer Ring zu höheren Werten der optischen Anisotropie. Polare Endgruppen $R^1$, wie Cyano, Halogen, Trifluormethyl oder Trifluormethoxy, oder ein Ring, wie Pyrimidin-2,5-diyl, erhöht z.B. die dielektrische Anisotropie. Laterale Halogen-Substituenten ergeben einen Beitrag zur Dielektrizitätskonstante sowohl parallel als auch senkrecht zur Moleküllängsachse, was je nach Substitutionsmuster zur Erhöhung oder Verminderung der dielektrischen Anisotropie oder zur Erzielung einer negativen dielektrischen Anisotropie ausgenützt werden kann. Ferner kann durch laterale Substituenten an einem oder mehreren Ringen eine allfällige Tendenz zur Ausbildung hochgeordneter smektischer Phasen meistens weitgehend unterdrückt und oft auch die Löslichkeit verbessert werden. Weiterhin können durch eine C=C-Doppelbindung in der Seitenkette die elastischen Eigenschaften, die Schwellenspannungen, die Ansprechzeiten, die Mesophasen usw. modifiziert werden.

Die vorliegende Erfindung ermöglicht daher neben der Induzierung einer hohen Verdrillung zusätzlich die Optimierung flüssigkristalliner, optischer und elektrooptischer Eigenschaften in einem weiten Bereich je nach Anwendung und gewünschten Eigenschaften.

Der Ausdruck "unsubstituiertes oder einfach oder mehrfach substituiertes 1,4-Phenylen, in welchem gegebenenfalls 1 oder 2 CH-Gruppen durch Stickstoff ersetzt sind", umfasst Gruppen wie 1,4-Phenylen, 2-Fluor-1,4-Phenylen, 2,3-Difluor-1,4-phenylen, 2-Chlor- 1,4-phenylen, 2-Chlor-1,4-phenylen, 2-Brom-1,4-phenylen, 2,3-Dicyano-1,4-phenylen, 2,3-Dicyano-1,4-phenylen, Pyridin-2,5-diyl, Pyrazin-2,5-diyl, Pyrimidin 2,5-diyl und dergleichen. Bevorzugte Gruppen sind 1,4-Phenylen, 2-Fluor-1,4-phenylen, 2,3-Difluor-1,4-phenylen, Pyridin-2,5-diyl, Pyrimidin-2,5-diyl und dergleichen.

Der Ausdruck "unsubstituiertes, einfach oder mehrfach durch Fluor oder Chlor substituiertes Alkyl oder Alkenyl mit 1 bzw. 2 bis 12 Kohlenstoffatomen, in welchem auch eine oder zwei nicht benachbarte $CH_2$-Gruppen durch -O- ersetzt sein können" umfasst im Rahmen der vorliegenden Erfindung geradkettige und verzweigte (gegebenenfalls chirale) Reste wie Alkyl, lE-Alkenyl, 3E-Alkenyl, 4-Alkenyl, Alkenyl mit endständiger Doppelbindung, Alkoxy, 2E-Alkenyloxy, 3-Alkenyloxy, Alkenyloxy mit endständiger Doppelbindung, Alkoxyalkyl, Alkenyloxyalkyl, 1-Fluoralkyl, 2-Fluoralkyl, 2-Fluoralkoxy, 1-Chloralkyl, 2-Chloralkyl, 2-Chloralkoxy, 1-Methylalkyl, 2-Methylalkyl, 1-Methylalkoxy, 2-Methylalkoxy und dergleichen. Beispiele bevorzugter Reste sind Methyl, Ethyl, Propyl, Butyl, Pentyl, Hexyl, Heptyl, Octyl, Nonyl, Decyl, Undecyl, Dodecyl, 1-Methylpropyl, 1-Methylheptyl, 2-Methylbutyl, 3-Methylpentyl, Vinyl, 1E-Propenyl, 1E-Butenyl, 1E-Pentenyl, 1E-Hexanyl, 1E-Heptenyl, 3-Butenyl, 3E-Pentenyl, 3E-Hexenyl, 3E-Heptenyl, 4-Pentenyl, 4Z-Hexenyl, 4Z-Heptenyl, 5-Hexenyl, 6-Heptenyl, 7-Octenyl, 8-Nonenyl, 9-Decenyl, 10-Undecenyl, 11-Dodecenyl, Methoxy, Ethoxy, Propyloxy, Butyloxy, Pentyloxy, Hexyloxy, Heptyloxy, Octyloxy, Nonyloxy, Decyloxy, Undecyloxy, Dodecyloxy, 1-Methylpropyloxy, 1-Methylheptyloxy, 2-Methylbutyloxy, 3-Methylpentyloxy, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, 7-Octenyloxy, 8-Nonenyloxy, 9-Decenyloxy, 10-Undecenyloxy, 11-Dodecenyloxy, Methoxymethyl, Ethoxymethyl, Propyloxymethyl, Allyloxymethyl, 1-Fluorpropyl, 1-Fluorpentyl, 1-Chlorpropyl,

2-Fluorpropyl, 2-Fluoropentyl, 2-Chlorpropyl, 2-Fluorpropyloxy, 2-Fluorbutyloxy, 2-Fluorpentyloxy, 2-Fluorhexyloxy, 2-Chlorpropyloxy, Chlorbutyloxy und dergleichen.

"Alkyl" in den Resten $R^2$, $R^3$, $R^4$ und $R^5$ bedeutet geradkettige und verzweigte, gegebenenfalls chirale Gruppen mit 1 bis 12 Kohlenstoffatomen, wie Methyl, Ethyl, Propyl, Isopropyl, Butyl, Isobutyl, 2-Butyl (= 1-Methylpropyl), 2-Methylbutyl, Pentyl, Hexyl, Heptyl, Octyl, 2-Octyl (= 1-Methylheptyl), und dergleichen. Bevorzugte Alkylreste in $R^2$, $R^3$, $R^4$ und $R^5$ sind $C_1$-$C_5$-Alkylreste, insbesondere jedoch Methyl, Ethyl, n-Propyl, n-Butyl und n-Pentyl.

Bevorzugte Verbindungen der Formel I sind diejenigen, worin n = 1 ist. Weiter sind diejenigen Verbindungen der Formel I bevorzugt, worin $X^1$ und $X^2$ oder $X^3$ und $X^4$ Fluor bedeuten. Bei ganz besonders bevorzugten Verbindungen der Formel I bedeutet $R^1$ Alkyl, Alkenyl, Alkoxy, Alkenyloxy, Alkoxyalkyl oder Alkoxyalkenyl mit 1 bzw. 2 bis 6 Kohlenstoffatomen.

Insbesondere sind auch diejenigen Verbindungen der Formel I bevorzugt, worin $R^1$ eine Gruppe der Formel

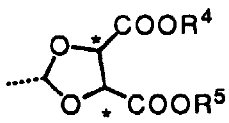

(a)

bedeutet und n = 0 oder 1 ist.

Besonders bevorzugter Verbindungen der Formel I sind die somit optisch aktiven Verbindungen der folgenden Formeln

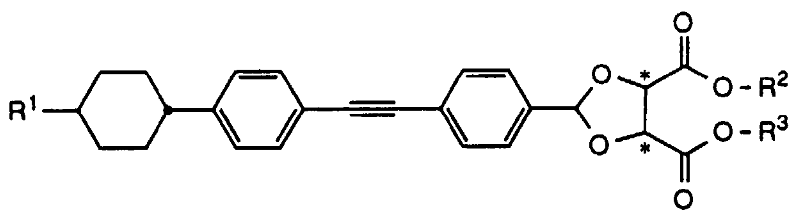

Ia

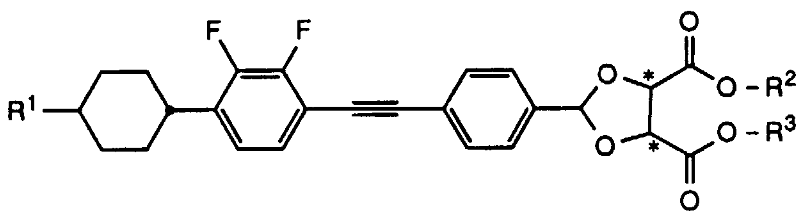

Ib

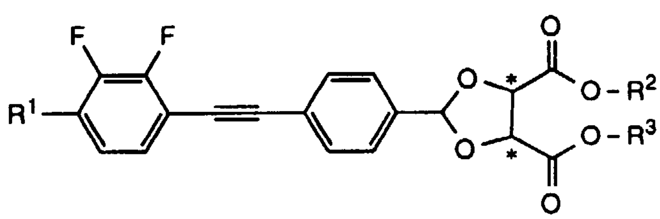

Ic

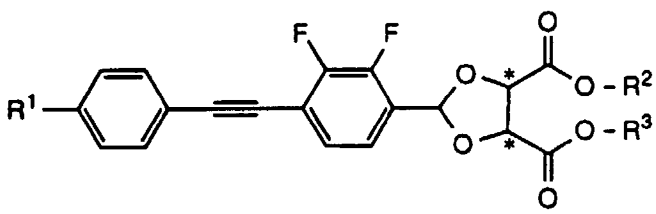

Id

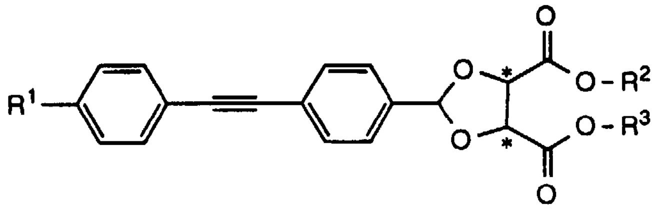

Ie

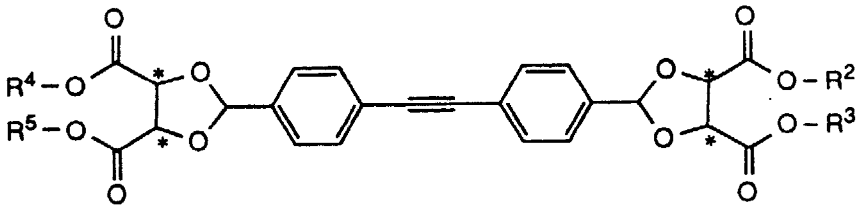

If

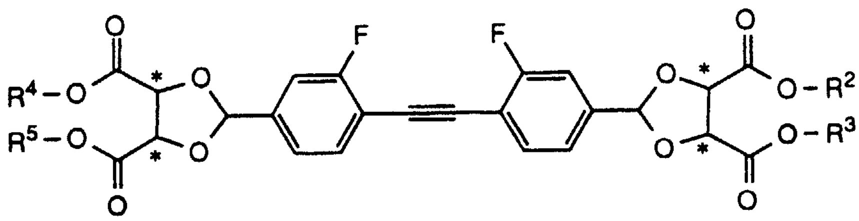

Ig

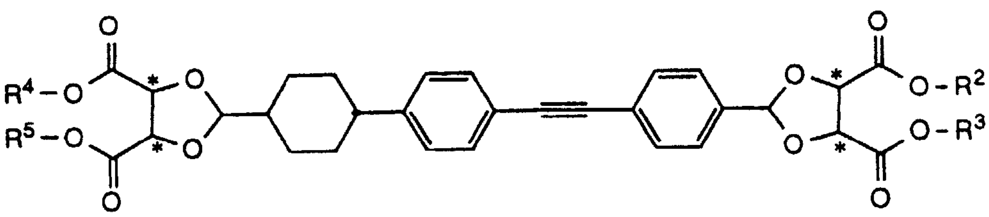

Ih

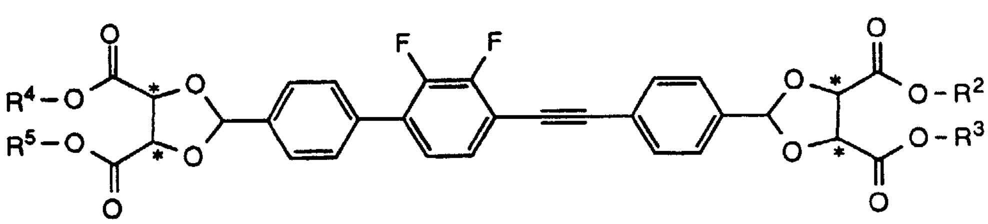

I i

worin $R^1$, Wasserstoff, unsubstituiertes, einfach oder mehrfach durch Fluor oder Chlor substituiertes Alkyl oder Alkenyl mit 1 bzw. 2 bis 12 Kohlenstoffatomen, in welchem auch eine $-CH_2$-Gruppe durch -O- ersetzt sein kann, oder - falls $R^1$ an einen aromatischen Ring gebunden ist - auch Fluor, Chlor, Cyano, $-CF_3$, $-OCHF_2$ oder $-OCF_3$ bedeutet; und $R^2$, $R^3$, $R^4$ und $R^5$ die oben angegebenen Bedeutungen haben.

Von den Verbindungen der Formeln If bis Ii sind diejenigen bevorzugt, worin $R^2$ gleich $R^4$ und $R^3$ gleich $R^5$ ist. Ganz besonders bevorzugt sind diejenigen Verbindungen der Formeln If bis Ii, worin $R^2$ und $R^3$ gleich $R^4$ und $R^5$ sind.

Die erfindungsgemässen Verbindungen können leicht in an sich bekannter Weise hergestellt werden. Dazu sind Acetylenkupplungen, wie sie für die Herstellung von Tolanen generell verwendet werden, besonders geeignet. So kann beispielsweise ein Phenylacetylen Derivat <u>3</u> mit einem optisch aktiven Phenyldioxolan <u>2</u>, welches in para Stellung einen geeigneten Substituenten, wie beispielsweise Jod, Brom oder Trifluormethylsulfonat (OTf) als Abgangsgruppe trägt, mit Hilfe eines geeigneten Palladiumkatalysators gekuppelt werden (Schema 1).

## Schema 1

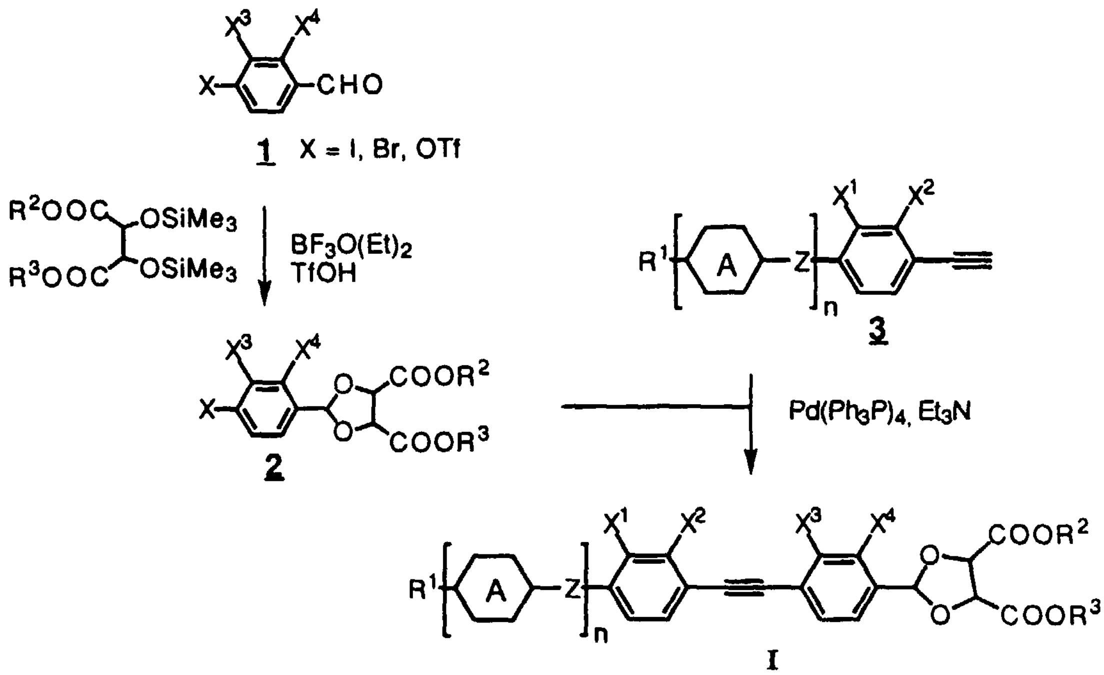


Diese Methode eignet sich insbesondere zur Herstellung von Verbindungen der Formel Ia bis Ie. Auch Verbindungen der Formel I, worin $R^1$ einen Dioxolanring bedeutet, in welchem $R^4$ und $R^5$ von $R^2$ und $R^3$ verschieden sind, können auf diese Weise hergestellt werden. Die Methode ist in Schema 2 weiter ausgeführt.

Schema 2

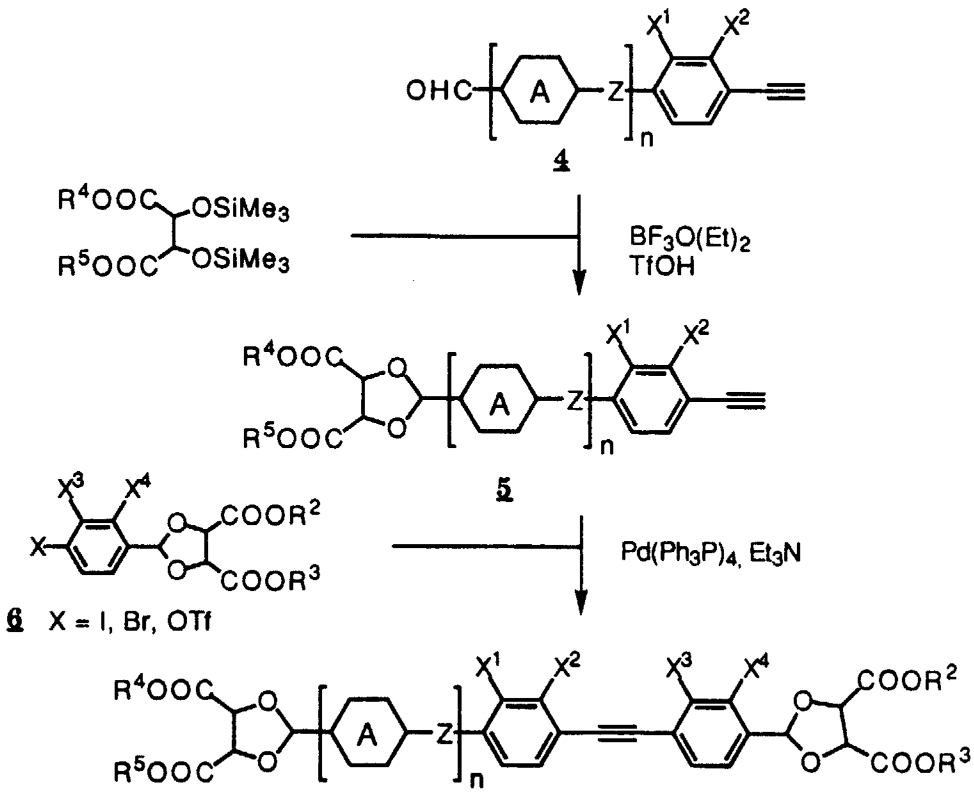


Verbindungen der allgemeinen Formel Ia bis Ie, oder Verbindungen der Formeln If bis Ii, worin $R^2$ gleich $R^4$ und $R^3$ gleich $R^5$ ist, können zweckmässig wie in Schema 3 aufgezeichnet, hergestellt werden. Dabei wird der Dioxolanring bzw. werden beide Dioxolanringe am Schluss der Synthese durch Acetalisierung eines Mono- bzw. Dialdehydes mit den gewünschten Weinsäurediester gebildet.

Schema 3

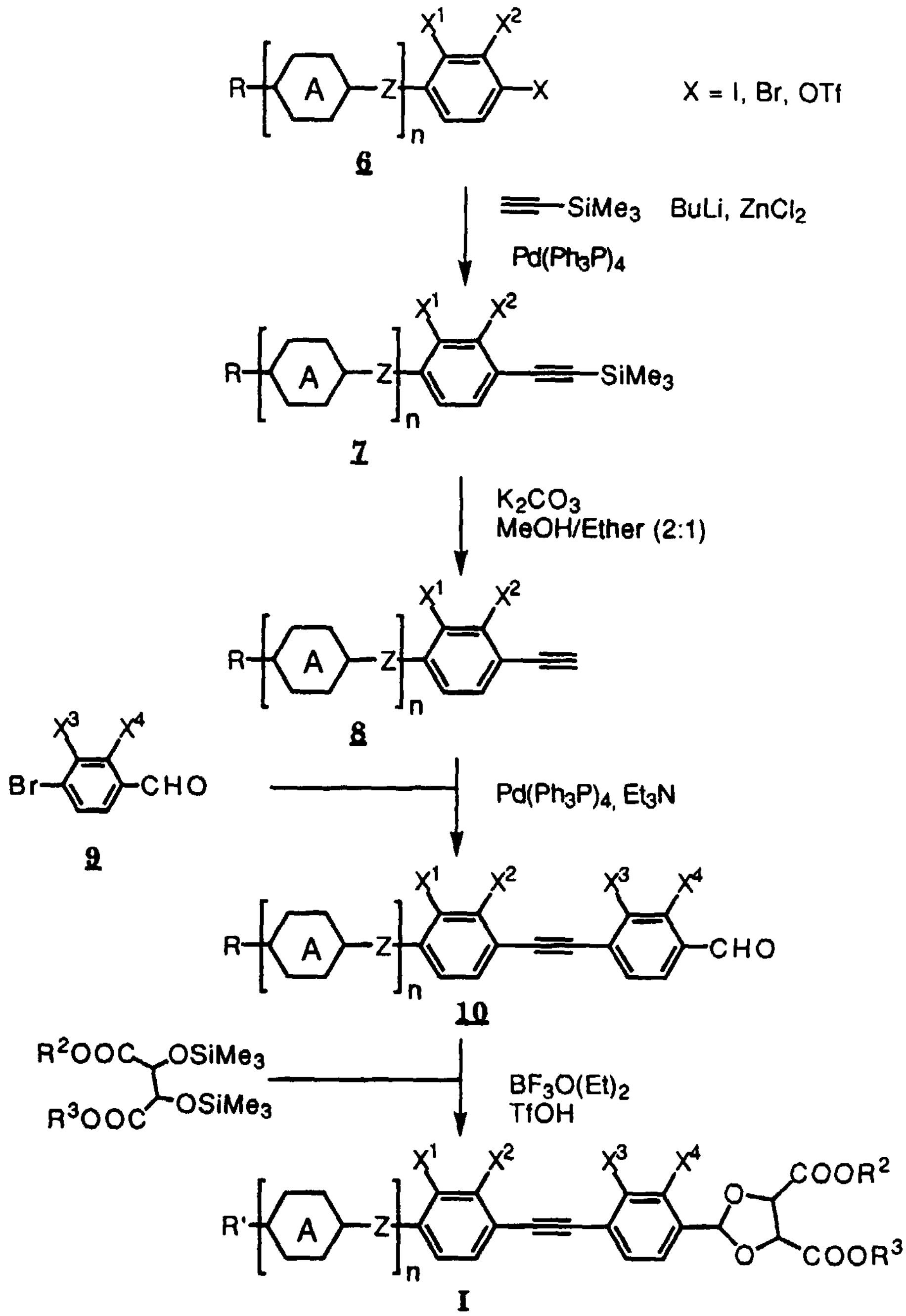


Die als Ausgangsmaterialien in den Schemen 1 und 2 aufgeführten Acetylenderivate sind bekannt oder Analoge bekannter Verbindungen. Ihre Herstellung ist in der Literatur mehrmals beschrieben worden, beispielsweise in EP-A 501 268 oder EP-A Nr. 593 997. Zudem sind sie gemäss Schema 3 in an sich bekannter Weise durch Kupplung mit Trimethylsilylacetylen und anschliessender Hydrolyse herstellbar.

Die Bildung des Dioxolanrings geschieht durch Acetalisierung des Ausgangsaldehyds mit einem geeigneten Weinsäurediester, bevorzugt in silylierter Form. Analoge Reaktionen sind beispielsweise in THL 21, 1357 (1980) beschrieben.

Bei genügend stabilen Aldehyden kann die Acetalisierung zum Dioxolan auch mit Bortrifluoridetherat ausgeführt werden.

Die Erfindung betrifft ebenfalls flüssigkristalline Gemische, enthaltend ein flüssigkristallines Trägermaterial und eine oder mehrere optisch aktive Verbindungen der Formel I. Als Trägermaterial eignen sich grundsätzlich alle Flüssigkristallmaterialien, welche eine verdrillbare Flüssigkristallphase mit einem adäquaten Mesophasenbereich auf-

weisen. Besonders geeignet sind die Verbindungen der Formel I als chirale Dotierstoffe für nematische oder cholesterische Trägermaterialien. Das flüssigkristalline Trägermaterial kann eine Einzelverbindung oder ein Gemisch sein und besitzt vorzugsweise einen Klärpunkt von mindestens etwa 60°C.

Der Anteil an chiralem Dotierstoff der Formel I ist im wesentlichen durch dessen Verdrillungsvermögen und die gewünschte Ganghöhe bestimmt. Der Anteil an chiralem Dotierstoff kann daher je nach Anwendung in einem breiten Bereich variieren und beispielsweise etwa 0,1-30 Gew. % betragen. Für Anzeigevorrichtungen auf der Basis von Flüssigkristallen mit verdrillt nematischer Struktur ist je nach Zellentyp und -dicke meist eine Ganghöhe von etwa 3-40 µm erforderlich und daher ein entsprechend kleiner Anteil ausreichend. Demgegenüber werden für Anwendungen, welche auf der Reflektion von sichtbarem Licht durch cholesterische Schichten beruhen, Ganghöhen von weniger als 2 µm, beispielsweise etwa 0,4-0,6 µm benötigt, was einen entsprechend höheren Anteil an chiralem Dotierstoff erfordert.

Geeignete flüssigkristalline Trägermaterialien sind in grosser Zahl bekannt und im Handel erhältlich. In der Regel sind flüssigkristalline Gemische enthaltend 2 oder mehrere Komponenten als Trägermaterialien bevorzugt. Grundsätzlich kann aber auch eine flüssigkristalline Verbindung als Trägermaterial verwender werden, wenn sie eine ausreichend breite Mesophase aufweist.

Vorzugsweise enthalten die erfindungsgemässen Gemische neben einer oder mehreren Verbindungen der Formel I eine oder mehrere Verbindungen aus der Gruppe der Verbindungen der allgemeinen Formeln

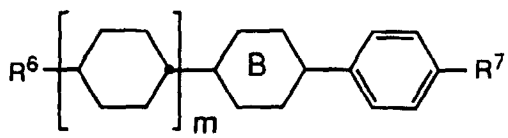

II

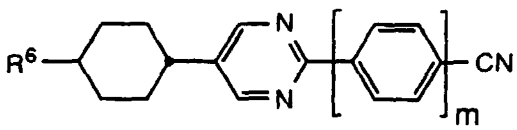

III

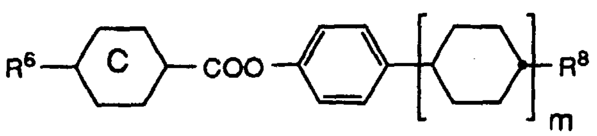

IV

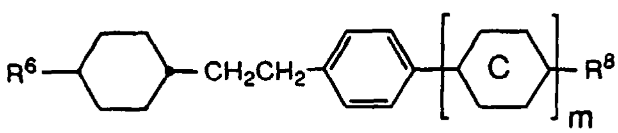

V

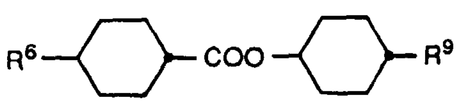

VI

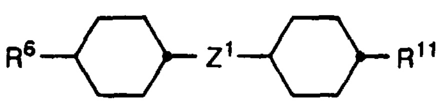

VII

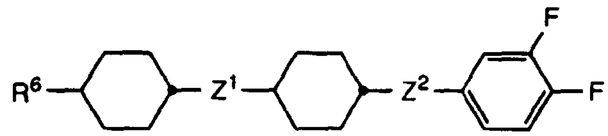

VIII

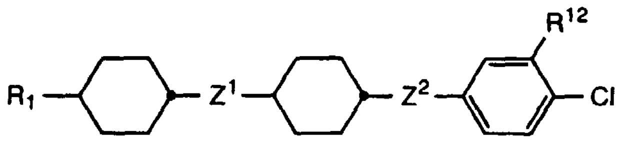

IX

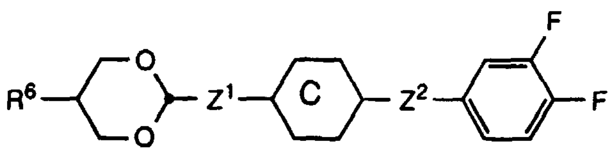

X

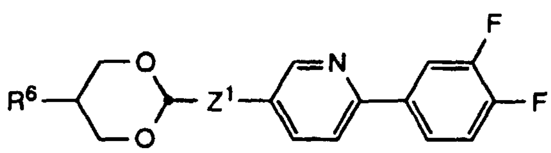

XI

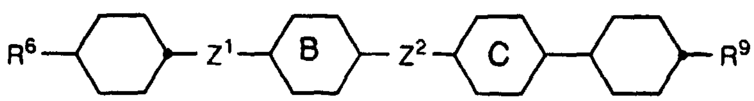

XII

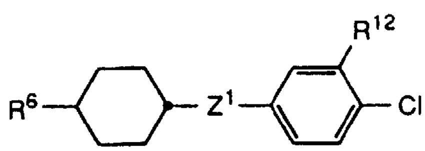

XIII

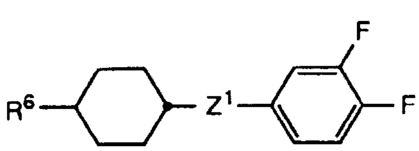

XIV

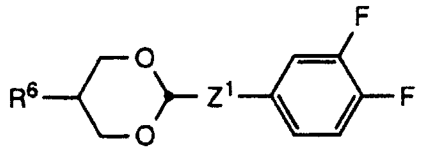

XV

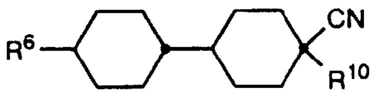

XVI

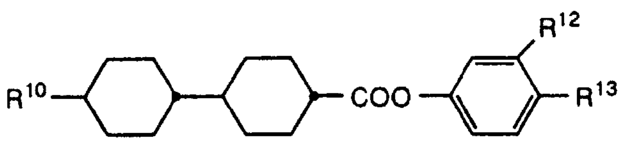

XVII

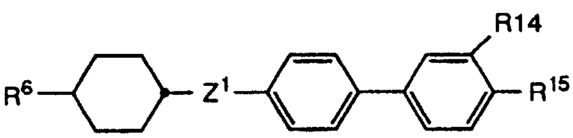

XVIII

worin

$R^6$,$R^9$ Alkyl, Alkoxyalkyl, 3E-Alkenyl, 4-Alkenyl oder an gesättigten Ringen auch 1E-Alkenyl bedeuten;

m 0 oder 1 bedeutet;

Ring B 1,4-Phenylen, Pyridin-2,5-diyl, Pyrimidin-2,5-diyl, trans-1,4-Cyclohexylen oder trans-1,3-Dioxan-2,5-diyl bedeutet;

$R^7$ Cyano, Isothiocyanato, Fluor, Alkyl, 3E-Alkenyl, 4-Alkenyl, Alkoxy, 2E-Alkenyloxy, 3-Alkenyloxy oder 1-Alkinyl darstellt;

Ring C 1,4-Phenylen oder trans-1,4-Cyclohexylen bedeutet;

$R^8$ Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy darstellt;

$R^{10}$ Alkyl, 1E-Alkenyl, 3E-Alkenyl oder 4-Alkenyl bedeutet;

$R^{11}$ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl darstellt;

$Z^l$, $Z^2$ unabhängig voneinander eine einfache Kovalenzbindung oder $-CH_2CH_2-$ bedeuten, wobei zwei aromatische Ringe immer durch eine einfache Kovalenzbindung verknüpft sind;

$R^{12}$ Wasserstoff, Fluor oder Chlor bedeutet;

$R^{13}$ Cyano, Fluor oder Chlor darstellt;

$R^{14}$ Wasserstoff oder Fluor darstellt; und

$R^{15}$ Fluor oder Chlor darstellt.

Der obige Ausdruck "gesättigter Ring" umfasst trans-1,4-Cyclohexylen und trans-1,3-Dioxan-2,5-diyl. Die Reste $R^6$ bis $R^{11}$ besitzen vorzugsweise je 1 bzw. 2 bis 12 Kohlenstoffatome, besonders bevorzugt je 1 bzw. 2 bis 7 Kohlenstoffatome. Geradkettige Reste sind im allgemeinen bevorzugt.

Der Ausdruck "Alkyl" bedeutet in diesem Zusammenhang vorzugsweise geradkettige Reste mit 1 bis 12 Kohlenstoffatomen, vorzugsweise mit 1 bis 7 Kohlenstoffatomen, wie beispielsweise Methyl, Ethyl, Propyl, Butyl, Pentyl, Hexyl oder Heptyl.

Der Ausdruck "Alkyloxyalkyl" bedeutet in diesem Zusammenhang vorzugsweise geradkettige Reste mit 1 bis 12 Kohlenstoffatomen, besonders bevorzugt mit 1 bis 7 Kohlenstoffatomen, wie beispielsweise Methoxymethyl, Ethoxymethyl, Propyloxymethyl, Butyloxymethyl, Methoxypropyl und dergleichen.

Der Ausdruck "Alkyloxy" bedeutet in diesem Zusammenhang vorzugsweise geradkettige Reste mit 1 bis 12 Kohlenstoffatomen, besonders bevorzugt mit 1 bis 7 Kohlenstoffatomen, wie beispielsweise Methoxy, Ethoxy, Propyloxy, Butyloxy, Pentyloxy, Hexyloxy, Heptyloxy und dergleichen.

Der Ausdruck "1E-Alkenyl" bedeutet in diesem Zusammenhang vorzugsweise geradkettige Alkenylreste mit 2 bis 12, besonders bevorzugt mit 2 bis 7 Kohlenstoffatomen, in denen die Doppelbindung sich in 1-Stellung befindet, wie beispielsweise Vinyl, 1E-Propenyl, 1E-Butenyl, 1E-Pentenyl, 1E-Hexenyl, 1E-Heptenyl und dergleichen.

Der Ausdruck "3E-Alkenyl" bedeutet in diesem Zusammenhang vorzugsweise geradkettige Alkenylreste mit 4 bis 12, besonders bevorzugt mit 4 bis 7 Kohlenstoffatomen, in denen die Doppelbindung sich in 3-Stellung befindet, wie beispielsweise 3-Butenyl, 3E-Pentenyl, 3E-Hexenyl, 3E-Heptenyl und dergleichen.

Der Ausdruck "4-Alkenyl" bedeutet in diesem Zusammenhang vorzugsweise geradkettige Alkenylreste mit 5 bis 12 Kohlenstoffatomen, in denen die Doppelbindung sich in 4-Stellung befindet, wie beispielsweise 4-Pentenyl, 4-Hexenyl, 4-Heptenyl und dergleichen.

Der Ausdruck "2E- bzw. 3Z-Alkenyloxy" bedeutet in diesem Zusammenhang vorzugsweise geradkettige Alkenyloxyreste mit 3 bzw. 4 bis 12 Kohlenstoffatomen, besonders bevorzugt mit 3 bzw. 4 bis 7 Kohlenstoffatomen, in denen die Doppelbindung sich in 2- bzw. 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy und dergleichen.

Der Ausdruck "1-Alkinyl" bedeutet in diesem Zusammenhang vorzugsweise geradkettige Alkinylreste mit 2 bis 12, besonders bevorzugt mit 2 bis 7 Kohlenstoffatomen, in denen die Dreifachbindung sich in 1-Stellung befindet, wie beispielsweise Ethinyl, 1-Propinyl, 1-Butinyl, 1-Pentinyl und dergleichen.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. Im Zusammenhang mit Flüssigkristallphasen und Phasenübergängen bedeuten C eine kristalline Phase, $S_B$ eine smektische B Phase, N eine nematische Phase, N* eine cholesterische Phase und I die isotrope Phase. Die Helixganghöhe wird mit p bezeichnet und die Wellenlänge des selektiv reflektierten, zirkular polarisierten Licht mit $\lambda_{max}$. Optische Antipoden besitzen jeweils "spiegelbildliche Eigenschaften", d.h. gleiche Schmelzpunkte etc., führen aber zu entgegengesetztem Helixdrehsinn und entgegengesetzter Zirkularpolarisation des reflektierten Lichts.

Beispiel 1

a) Zu 4 ml Ethinyltrimethylsilan in 50 ml wasserfreiem Tetrahydrofuran wurden unter Stickstoff bei -50°C 18,8 ml Buthyllithium in Hexan zugetropft, und nach 30 Min. 57 ml 1M etherische Zinkchloridlosung zugetropft. Hierauf wurde die Kühlung entfernt und während 1 Std. ausreagieren gelassen. Dann wurde auf 0°C abgekühlt und zur Reaktionslösung nacheinander 5 g 4-(trans-4-propylcyclohexyl)-phenyltrifluormethylsulfonat in 50 ml Dimethylformamid, 0,824 g Tetrakis(triphenylphosphin)palladium(0) und 1,2 g Lithiumchlorid zugefügt. Das Reaktionsgemisch wurde auf 85°C erhitzt und bei dieser Temperatur über Nacht reagieren gelassen. Zur Vervollständigung der Reaktion wurde hierauf nochmals eine gleiche Menge der wie oben hergestellten Lösung von Zinkethinyltrimethylsilan-Lösung zugefügt und weiter bei 85°C reagieren gelassen. Nach einer Stunde wurde abgekühlt, mit 20 ml Wasser versetzt, zwischen 1N Schwefelsäure und Pentan verteilt und die abgetrennte organische Phase mit Wasser gewaschen. Die organische Phase wurde über Magnesiumsulfat getrocknet, über Celite filtriert und vollständig eingedampft. Dies ergab 3,82 g 1[-4-(trans-4-propylcyclohexyl)phenyl]-2-trimethylsilylacetylen als feste Masse (GC Reinheit 97%), welches direkt in der nächsten Stufe eingesetzt wurde.

b) Eine Mischung von 3,8 g 1-[4-(trans-4-propylcyclohexyl)phenyl]-2-trimethylsilylacetylen, 0,4 g Kaliumcarbonat und 50 ml Methanol/Ether (2:1) wurde während 1 Std. bei Raumtemperatur gerührt. Dann wurde zwischen Wasser und Ether verteilt, die organische Phase nacheinander mit gesättigter Kochsalzlösung und Wasser gewaschen, über Magnesiumsulfat getrocknet, über Celite filtriert und eingedampft. Nach Chromatographie des Rückstandes über 150 Kieselgel mit Cyclohexan wurden 1,95 g 4-(trans-4-Propylcyclohexyl)phenylacetylen als farbloser Festkörper erhalten.

c) Zu einer Lösung von 5g 4-Brombenzaldehyd und 13,2 g (2R,3R)-2,3-Bis-trimethylsilyloxy-glutarsäure-dibutylester wurden bei 0°C 0,54 ml Bortrifluorid-etherat getropft und dann während 30 Min. reagieren gelassen. Danach wurden noch immer bei 0°C 0,24 ml Trifluormethansulfonsäure zugegeben und 30 Min. reagieren gelassen. Dann wurde das Kühlbad entfernt und das Reaktionsgemisch während 4 Std. bei Raumtemperatur gerührt. Anschliessend wurde die Reaktionslösung auf eine gesättigte wässrige Natriumbicarbonatlösung gegossen, zwischen Wasser und Methylenchlorid verteilt, die organische Phase abgetrennt, über Magnesiumsulfat getrocknet, über Celite filtriert und eingedampft. Chromatographie des Rückstandes an 250 g Kieselgel mit Essigester/Cyclohexan (1:9) ergab 9,3 g (4R, 5R)-2-(4-Bromphenyl)-1,3-dioxolan-4,5-dicarbonsäure-dibutylester (GC-Reinheit 98%).

d) Zu einer Lösung von 0,5 g 4-(trans-4-Propylcyclohexyl)phenylacetylen in 5 ml trockenem Tetrahydrofuran wurden bei -50°C 1,45 ml ca. 1,6 M Butyllithiumlösung in Hexan getropft und während 30 Min. bei dieser Temperatur reagieren gelassen. Dann wurden 4,4 ml einer 1 M Zinkchlorid Lösung in Ether zugetropft, das Kühlbad entfernt und während 30 Min. reagieren gelassen. Hierauf wurde auf 0°C abgekühlt und bei dieser Temperatur eine Lösung von 0,95 g (4R, 5R)-2-(4-Bromphenyl)-1,3-dioxolan-4,5-dicarbonsäure-dibutylester in 5 ml trockenem Tetrahydrofuran zugetropft und danach 0,128 g Tetrakis(triphenylphosphin)palladium(0) zugefügt. Das Reaktionsgemisch wurde dann während 15 Std. auf 60°C erhitzt, dann abgekühlt, zwischen Wasser und Ether verteilt, die organische Phase abgetrennt und über Magnesiumsulfat getrocknet. Hierauf wurde über Celite filtriert, das Lösungsmittel abgedampft und an 100 g Kieselgel mit 5% Essigester in Cyclohexan chromatographiert. Weitere Reinigung mit Aktivkohle in Isopropanol und zweimalige Kristallisation aus Isopropanol ergab 0,23 g (4R, 5R)-2-(4-[4-(trans-4-Propylcyclohexyl)phenyläthinyl]phenyl}-1,3-dioxolan-4,5-dicarbonsäure-dibutylester, Smp. (K-N*) 74,9°C, Klp. (N*-I) 78,3°C.

Auf analoge Weise können die folgenden Verbindungen hergestellt werden:

(4R, 5R)-2-{4-[4-(trans-4-Propylcyclohexyl)phenyläthinyl]phenyl}-1,3-dioxolan-4,5-dicarbonsäure-diethylester;

(4R, 5R)-2-{4-[4-(trans-4-Pentylcydohexyl)phenyläthinyl]phenyl}-1,3-dioxolan-4,5-dicarbonsäure-dibutylester;

(4R, 5R)-2-{4-[2,3-Difluor-4-(trans-4-propylcyclohexyl)phenyläthinyl]-phenyl}-1,3-dioxolan-4,5-dicarbonsäure-dibutylester;

(4R, 5R)-2-{2,3-Difluor4-[4-(trans-4-propylcyclohexyl)phenyläthinyl]phenyl}-1,3-dioxolan-4,5-dicarbonsäure-dibutylester, Smp. (K-I) 73,4°C, Klp. (N*-I) 68,2°C;

(4R, 5R)-2-{3-Fluor-4-[2-fluor-4-(trans-4-propylcyclohexyl)phenyläthinyl]phenyl}-1,3-dioxolan-4,5-dicarbonsäure-dibutylester;

(4R, 5R)-2-[4-(4-Propyl-phenyläthinyl)phenyl]-1,3-dioxolan-4,5-dicarbonsäure-dibutylester;

(4R, 5R)-2-{4-[4-(2,3-Difluor-4-propylphenyl)phenyl}-1,3-dioxolan-4,5-dicarbonsäure-dibutylester;

(4R, 5R)-2-{4-[4-(2,3-Difluor-4-pentylphenyl)phenyläthinyl]phenyl}-1,3-dioxolan-4,5-dicarbonsäure-dibutylester;

(4R, 5R)-2-{4-[2,3-Difluor-4-(4-propylphenyl)phenyläthinyl]phenyl}-1,3-dioxolan-4,5-dicarbonsäure-dibutylester;

(4R, 5R)-2-{4-[4-(2,3-Difluor-4-propyloxyphenyl)phenyläthinyl]phenyl}-1,3-dioxolan-4,5-dicarbonsäure-dibutylester;

(4R, 5R)-2-[4-(2,3-Difluor-4-propyl-phenyläthinyl)phenyl]-1,3-dioxolan-4,5-dicarbonsäure-dibutylester, Smp. (K-I) 35°C;

(4R, 5R)-2-[2,3-Difluor-4-(4-propyl-phenyläthinyl)phenyl]-1,3-dioxolan-4,5-dicarbonsäure-dibutylester, Smp. (K-I) 75,3°C;

(4R, 5R)-2-[3-Fluor-4-(2-fluor-4-propyl-phenyläthinyl)phenyl]-1,3-dioxolan-4,5-dicarbonsäure-dibutylester;

(4R, 5R)-2-[4-(2,3-Difluor-4-pentyl-phenyläthinyl)phenyl]-1,3-dioxolan-4,5-dicarbonsäure-dibutylester;

(4R, 5R)-2-[4-(2,3-Difluor-4-pentyl-phenyläthinyl)phenyl]-1,3-dioxolan-4,5-dicarbonsäure-diäthylester;

(4R, 5R)-2-{4-[4-(2-(trans-4-Propylcyclohexyl)äthyl)phenyläthinyl] phenyl}-1,3-dioxolan-4,5-dicarbonsäure-dibutylester;

(4R, 5R)-2-{4-[2,3-Difluor-4-(2-(trans-4-propylcyclohexyl)äthyl) phenyläthinyl]phenyl}-1,3-dioxolan-4,5-dicarbonsäure-dibutylester;

<u>Beispiel 2</u>

a) Ein Gemisch von 1 g 4-Ethinyl-benzaldehyd, 1,56 g 4-Brombenzaldehyd, 0,22 g Tetrakis(triphenylphosphin) palladium(0) und 10 ml Triäethylamin wurden während 16 Std. bei 80°C gerührt. Dann wurde das Reaktionsgemisch zwischen Wasser und Methylenchlorid verteilt, die organische Phase abgetrennt, über Magensiumsulfat getrocknet, filtriert und eingedampft. Dies ergab 1,54 g Tolan-4,4'-dicarboxaldehyd als festen bräunlichen Rückstand (GC-Reinheit 98,8%), welcher ohne weitere Reinigung für die folgende Stufe verwendet wurde.

b) Zu einem Gemisch von 2,6 g (2R,3R)-2,3-Bis-trimethylsilyloxyglutarsäure-dibutylester, 0,039 ml Trifluormethansulfonsäuretrimethylsilylester und 10 ml Methylenchlorid wurde eine Suspension von 0,5 g Tolan-4,4'-dicarboxaldehyd in 5 ml Methylenchlorid zugefügt und das Reaktionsgemisch während 4 Std. bei Raumtemperatur gerührt. Hierauf werden nochmals 0,04 ml Trifluormethansulfonsäure-trimethylsilylester hinzugefügt und während 64 Std. bei Raumtemperatur weiter gerührt. Nach Zugabe von 0,5 ml Pyridin wurde das Reaktionsgemisch zwischen Natriumbicarbonat und Methylenchlorid verteilt, die organische Phase abgetrennt, über Magnesiumsulfat getrocknet, über ein Gemisch von Celite/Alox (neutral, akt. III) filtriert und eingedampft. Chromatographie des Rückstandes an 100 g Kieselgel mit 5% Essigester in Toluol und anschliessende Kristallisation aus Hexan und dann aus Isopropanol ergab 0,88 g 4,4'-Bis-[(4R, 5R)-4,5-Dibutyloxycarbonyl-1,3-dioxolan-2-yl]tolan als weisse Kristalle, Smp. 54°C;

Auf analoge Weise können die folgenden Verbindungen hergestellt werden:

4,4'-Bis-[(4R, 5R)-4,5-Diethoxycarbonyl-1,3-dioxolan-2-yl]tolan;
4,4'-Bis-[(4R, 5R)-4,5-Dimethoxycarbonyl-1,3-dioxolan-2-yl]tolan;
2,3-Difluor-4,4'-bis-[(4R, 5R)-4,5-Dibutyloxycarbonyl-1,3-dioxolan-2-yl]tolan;
2,2'-Difluor-4,4'-bis-[(4R, 5R)-4,5-Dibutyloxycarbonyl-1,3-dioxolan-2-yl]tolan, Smp. (K-I) 70°C, Klp. monotrop (S*-I) 59,8°C;
4-{4-[(4R, 5R)-4,5-Diethoxycarbonyl-1,3-dioxolan-2-yl]phenyl}-4'-[(4R, 5R)-4,5-dibutyloxycarbonyl-1,3-dioxolan-2-yl]tolan;
4-{2,3-Difluor-4-[(4R, 5R)-4,5-diethoxycarbonyl-1,3-dioxolan-2-yl]phenyl}-4'-[(4R, 5R)-4,5-dibutyloxycarbonyl-1,3-dioxolan-2-yl]tolan;
2,3-Difluor-4-{4-[(4R, 5R)-4,5-dibutyloxycarbonyl-1,3-dioxolan-2-yl]phenyl}-4'-[(4R, 5R)-4,5-Dibutyloxycarbonyl-1,3-dioxolan-2-yl]tolan, Smp. (K-I) 73,1°C
4-{4-[(4R, 5R)-4,5-Diethoxycarbonyl-1,3-dioxolan-2-yl]phenyl}-2',3'-difluor-4'-[(4R, 5R)-4,5-Dibutyloxycarbonyl-1,3-dioxolan-2-yl]tolan;

<u>Beispiel 3</u>

Zur Messung der induzierten Ganghöhe (Pitch) und deren Temperaturabhängigkeit in Flüssigkristallmaterialien wurde die folgende Flüssigkristall-Grundmischung BM-1 verwendet:

| | |
|---|---|
| 5,36 Gew. % | 4'-Ethyl-4-cyanobiphenyl, |
| 3,18 " | 4'-Propyl-4-cyanobiphenyl, |
| 6,08 " | 4'-Butyl-4-cyanobiphenyl, |
| 6,53 " | 4-(trans-4-propylcyclohexyl)benzonitril, |
| 14,64 " | 4-(trans-4-Pentylcyclohexyl)benzonitril, |
| 5,21 " | 4-Ethyl-1-(trans-4-propylcyclohexyl)benzol, |
| 16,54 " | 4-Ethoxy-1-[2-(trans-e-propylcyclohexyl)-äthyl]benzol, |
| 5,60 " | 4"-Pentyl-4-cyano-p-terphenyl, |
| 5,71 " | 4'-(trans-4-Pentylcyclohexyl)-4-cyanobiphenyl, |
| 15,59 " | 1-[2-trans-4-Butylcyclohexyl)äthyl]-4-(trans-4-pentylcyclohexyl)benzol, |
| 4,74 " | 1-[2-trans-4-Butylcyclohexyl)äthyl]-4'-(trans-4-pentylcyclohexyl)biphenyl, |
| 7,59 " | 1-[2-trans-4-Butylcyclohexyl)äthyl]-4'-(trans-4-pentylcyclohexyl)- 1,1'-äthylendibenzol, |
| 2,84 " | 1-[2-trans-4-Propylcyclohexyl)äthyl]-cyclohexancarbonsäure-4-cyanophenylester; |

Flüssigkristall-Grundmischung BM-1 wurde jeweils mit 1,0 Gew.-% eines der folgenden optisch aktiven Dotierstoffe versetzt:

D-1 = 4R, 5R)-2-{4-[4-(trans-4-Propylcyclohexyl)phenyläthinyl]phenyl}-1,3-dioxolan-4,5-dicarbonsäure-dibutylester,

D-2 = (4R, 5R)-2-{2,3-Difluor4-[4-(trans-4-propylcyclohexyl)phenyläthinyl]-phenyl}-1,3-dioxolan-4,5-dicarbonsäure-dibutylester,

D-3 = (4R, 5R)-2-[4-(2,3-Difluor-4-propyl-phenyläthinyl)phenyl]-1,3-dioxolan-4,5-dicarbonsäure-dibutylester,

D-4 = (4R, 5R)-2-[2,3-Difluor-4-(4-propyl-phenyläthinyl)phenyl]-1,3-dioxolan-4 ,5-dicarbonsäure-dibutylester,

D-5 = 4,4'-Bis-[(4R, 5R)-4,5-Dibutyloxycarbonyl-1,3-dioxolan-2-yl]tolan,

D-6 = 4,4'-Bis-[(4S,5S)-4,5-Dibutyloxycarbonyl-1,3-dioxolan-2-yl]tolan,

D-7 = 2,2'-Difluor-4,4'-bis-[(4R,5R)-4,5-dibutyloxycarbonyl-1,3-dioxolan-2-yl]tolan,

D-8 = 2,3-Difluor-4-(4-[(4R,5R)-4,5-dibutyloxycarbonyl-1,3-dioxolan-2-yl]-phenyl)-4'-[(4R,5R)-4,5-dibutyloxycarbonyl-1,3-dioxolan-2-yl]tolan.

Für die chiral dotierten Gemische wurden die in Tabelle 1 zusammengestellten Ergebnisse erhalten, wobei A, B und C die Parameter der Reihenentwicklung

$$\frac{1}{pc} = A + BT_1 + CT_1^2$$

bezeichnen und p, c und $T_1$ die folgenden Bedeutungen haben:

$T_1$ = T-22°C

T = Temperatur in °C

p = Ganghöhe in mm (ein positiver Wert bedeutet rechtsdrehende Helixstruktur und ein negativer Wert bedeutet linksdrehende Helixstruktur)

c = Konzentration des optisch aktiven Dotierstoffes in Gew.-%.

Tabelle 1

| Gemessene Parameter von 1 Gew.-% des jeweiligen Dotierstoffes in der Basismischung BM-1 bei 22°C | | | | |
|---|---|---|---|---|
| Dotierstoff | A [$10^{-2}$·$mm^{-1}$·Gew.$\%^{-1}$] | B [$10^{-4}$·$mm^{-1}$·Gew.$\%^{-1}$] | C [$10^{-6}$·$mm^{-1}$·Gew.$\%^{-1}$] | p.c(22°C) [mm·Gew-%] |
| D-1 | -16,9 | 4,99 | -1,84 | -5,90 |
| D-2 | -10,5 | -0,77 | -0,61 | -9,50 |
| D-3 | -17,2 | 7,40 | -3,30 | -5,81 |
| D-4 | -11,9 | -0,34 | -1,51 | -8,38 |
| D-5 | -26,1 | 8,39 | -4,52 | -3,83 |
| D-6 | 25,7 | -7,93 | 2,77 | 3,89 |
| D-7 | -18,0 | 2,0 | -0,76 | -5,57 |
| D-8 | -22,1 | 2,70 | -0,70 | -4,53 |

**Patentansprüche**

1. Optisch aktive Verbindungen der allgemeinen Formel:

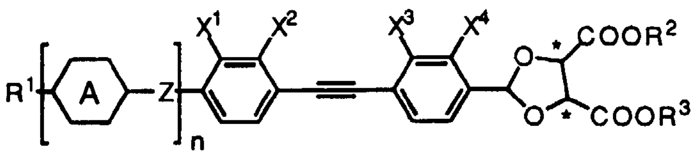

I

worin

Ring A unsubstituiertes oder einfach oder mehrfach substituiertes 1,4-Phenylen, in welchem gegebenenfalls eine oder zwei CH-Gruppen durch Stickstoff ersetzt sein können, oder trans-1,4-Cyclohexylen bedeutet;

Z eine einfache Kovalenzbindung, $-CH_2CH_2-$, $-CH_2O-$oder $-OCH_2-$ bedeutet;

$X^1$, $X^2$, $X^3$, $X^4$ unabhängig voneinander Wasserstoff oder Fluor darstellen;

$R^1$ Wasserstoff, unsubstituiertes, einfach oder mehrfach mit Fluor oder Chlor substituiertes Alkyl oder Alkenyl mit 1 bzw. 2 bis 12 Kohlenstoffatomen, in welchen auch eine oder zwei nicht benachbarte $CH_2$-Gruppen durch -O- ersetzt sein können, oder eine Gruppe der Formel

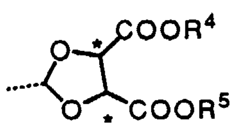

(a)

oder - falls $R^1$ an einen aromatischen Ring gebunden ist, auch Fluor, Chlor, Cyano, $-CF_3$, $-OCHF_2$ oder $-OCF_3$, bedeutet;

$R^2$, $R^3$, $R^4$, $R^5$ Alkyl oder Alkenyl mit 1 bzw. 2 bis 8 Kohlenstoffatomen bedeuten;

n 0 oder 1 bedeutet; und

* ein Chiralitätszentrum markiert.

2. Optisch aktive Verbindungen gemäss Anspruch 1, worin die Chiralitätszentren in 4- und 5- Stellung der Dioxolanringe beide die [R]- oder beide die [S]-Konfiguration aufweisen.

3. Optisch aktive Verbindungen gemäss einen der Ansprüche 1 oder 2, worin n = 1 ist.

4. Optisch aktive Verbindungen gemäss einen der Ansprüche 1 bis 3, worin $X^1$ und $X^2$ oder $X^3$ und $X^4$ Fluor bedeuten.

5. Optisch aktive Verbindungen gemäss einen der Ansprüche 1 bis 4, worin $R^1$ Alkyl, Alkenyl, Alkoxy, Alkenyloxy, Alkoxyalkyl oder Alkoxyalkenyl mit 1 bzw. 2 bis 6 Kohlenstoffatomen bedeuten.

6. Optisch aktive Verbindungen der Formeln

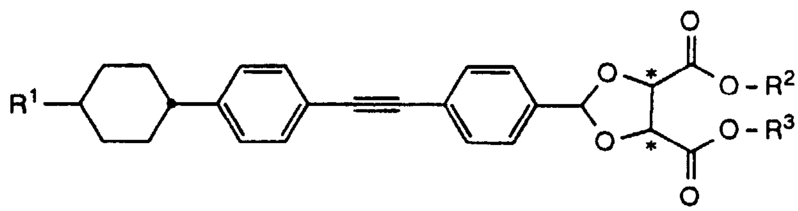

Ia

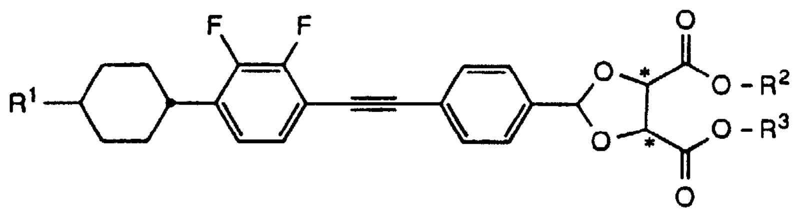

Ib

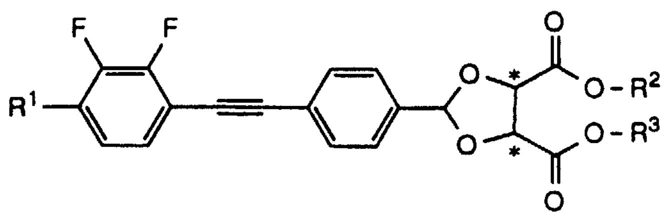

Ic

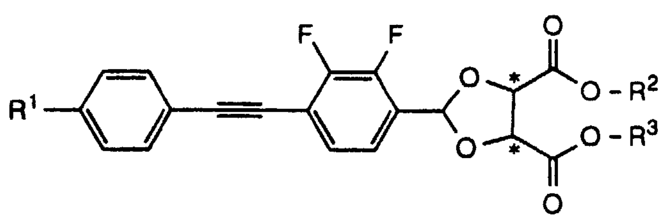

Id

und

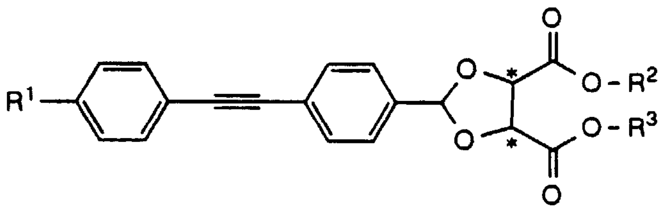

Ie

worin $R^1$ Wasserstoff, unsubstituiertes, einfach oder mehrfach mit Fluor oder Chlor substituiertes Alkyl oder Alkenyl mit 1 bzw. 2 bis 12 Kohlenstoffatomen, in welchem auch eine $-CH_2$-Gruppe durch -O- ersetzt sein kann, oder - falls $R^1$ an einen aromatischen Ring gebunden ist - auch Fluor, Chlor, Cyano, $-CF_3$, $-OCHF_2$ oder $-OCF_3$ bedeutet, und $R^2$ und $R^3$ die in Anspruch 1 angegebenen Bedeutungen haben.

**7.** Optisch aktive Verbindungen gemäss Anspruch 1, worin $R^1$ eine Gruppe der Formel

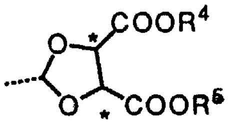

(a)

bedeutet und n = 0 oder 1 ist.

**8.** Flüssigkristallines Gemisch enthaltend mindestens eine Verbindung gemäss einem der Ansprüche 1 bis 7 als chiraler Dotierstoff.

**9.** Verwendung von optisch aktiven Verbindungen der in Anspruch 1 definierten allgemeinen Formel I für optische und elektro-optische Vorrichtungen.

**10.** Verwendung von flüssigkristallinen Gemischen nach Anspruch 8 für optische und elektrooptische Vorrichtungen.

**11.** Verfahren zur Herstellung von Verbindungen der allgemeinen Formel

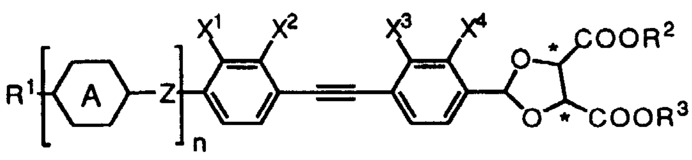

I

worin $R^1$, $R^2$, $R^3$, n, A, Z, $X^1$, $X^2$, $X^3$ und $X^4$ wie in Anspruch 1 definiert sind, dadurch gekenzeichnet, dass ein optisch aktives Dioxolan-Derivat der Formel

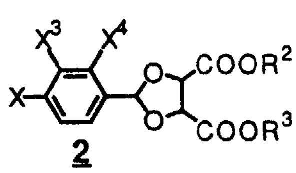

2

worin X eine Abgangsgruppe bedeutet und $X^3$ und $X^4$ die in Anspruch 1 angegebenen Bedeutungen haben, mit Hilfe eines geeigneten Palladiumkatalysators mit einem Acetylen Derivat der Formel

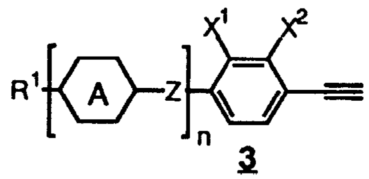

3

worin $R^1$, A, Z, $X^1$ und $X^2$ wie in Anspruch 1 definiert sind, gekoppelt wird.

## Claims

1. Optically active compounds of the general formula:

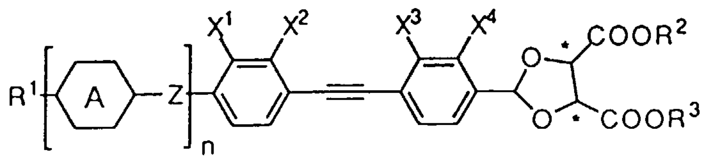

I

wherein

ring A signifies 1,4-phenylene, which is unsubstituted or mono- or multiply-substituted and in which optionally one or two CH-groups can be replaced by nitrogen, or signifies trans-1,4-cyclohexylene;

Z signifies a single covalent bond, $-CH_2CH_2-$, $-CH_2O-$or $-OCH_2-$;

$X^1,X^2,X^3,X^4$ each independently represent hydrogen or fluorine;

$R^1$ signifies hydrogen, alkyl or alkenyl with 1 or, respectively, 2 to 12 carbon atoms, which is unsubstituted or mono- or multiply-substituted with fluorine or chlorine and in which one or two non-adjacent $CH_2$-groups can be replaced by -O-, or a group of the formula

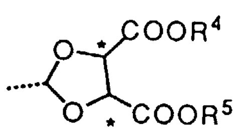

(a)

or - where $R^1$ is bonded to an aromatic ring - also fluorine, chlorine, cyano, $CF_3$, $OCHF_2$ or $-OCF_3$;

$R^2,R^3,R^4,R^5$ signify alkyl or alkenyl with 1 or, respectively, 2 to 8 carbon atoms;

n signifies 0 or 1; and

* marks a centre of chirality.

2. Optically active compounds according to claim 1, wherein the centres of chirality in the 4-position and 5-position of the dioxolane ring both have the [R] configuration or both have the [S] configuration.

3. Optically active compounds according to claim 1 or 2, wherein n = 1.

4. Optically active compounds according to any one of claims 1 to 3, wherein $X^1$ and $X^2$ or $X^3$ and $X^4$ signify fluorine.

5. Optically active compounds according to any one of claims 1 to 4, wherein $R^1$ signifies alkyl, alkenyl, alkoxy, alkenyloxy, alkoxyalkyl or alkoxyalkenyl with 1 or, respectively, 2 to 6 carbon atoms.

**6.** Optically active compounds of the formulae

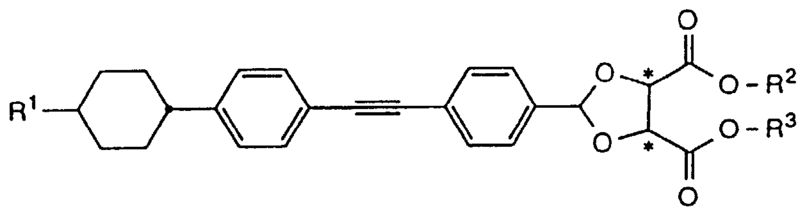

Ia

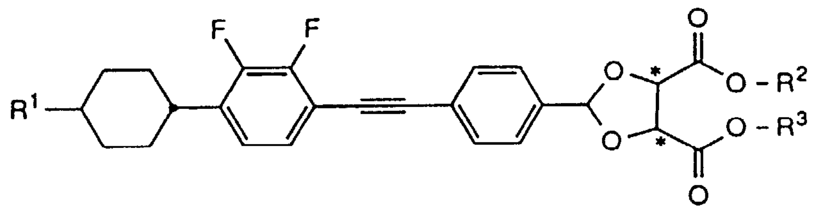

Ib

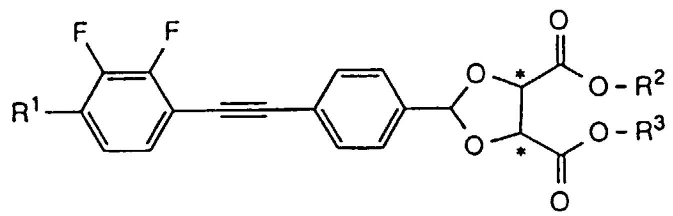

Ic

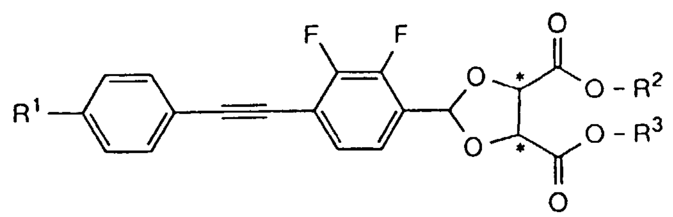

Id

and

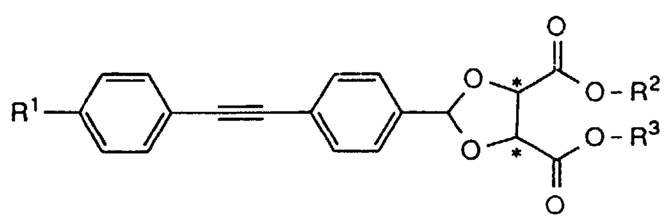

Ie

wherein $R^1$ signifies hydrogen, alkyl or alkenyl with 1 or, respectively, 2 to 12 carbon atoms, which is unsubstituted or mono- or multiply-substituted with fluorine or chlorine and in which one $-CH_2-$ group can be replaced by -O-, or - where $R^1$ is bonded to an aromatic ring - also fluorine, chlorine, cyano, $CF_3$, $-OCHF_2$ or $-OCF_3$, and $R^2$ and $R^3$ have the significances given in claim 1.

**7.** Optically active compounds according to claim 1, wherein $R^1$ signifies a group of the formula

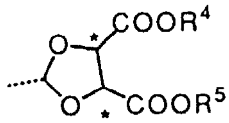

(a)

and n = 0 or 1.

**8.** A liquid crystalline mixture containing at least one compound in accordance with any one of claims 1 to 7 as a chiral dopant.

**9.** The use of optically active compounds of general formula I defined in claim 1 for optical and electro-optical devices.

**10.** The use of liquid crystalline mixtures according to claim 8 for optical and electro-optical devices.

**11.** A process for the manufacture of compounds of formula I

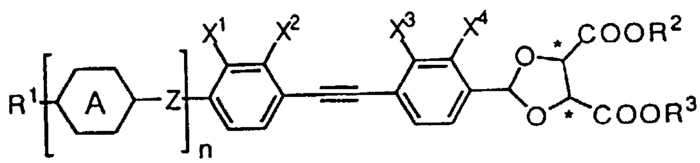

I

wherein $R^1$, $R^2$, $R^3$, n, A, Z, $X^1$,$X^2$,$x^3$ and $X^4$ are defined in claim 1, characterized in that an optically active dioxolane derivative of the formula

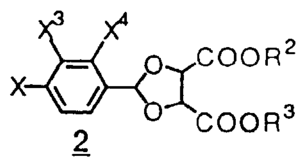

2

wherein X signifies a releasing group and $X^3$ and $X^4$ are defined in claim 1, will be coupled with an acetylene derivative of the formula

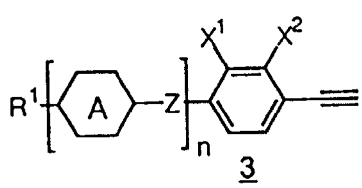

3

wherein $R^1$, A, Z $X^1$, $X^2$ are defined in claim 1 with the aid of a palladium catalyst.

## Revendications

**1.** Composés optiquement actifs de la formule générale :

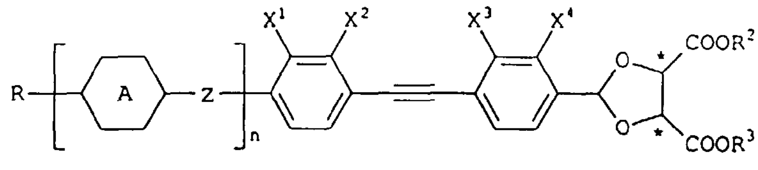

I

dans laquelle :

le cycle A est un groupe 1,4-phénylène non substitué ou bien unisubstitué ou plurisubstitué dans lequel un ou deux groupes CH sont le cas échéant remplacés par un azote, ou un groupe trans-1,4-cyclohexylène ;

Z est une liaison covalente simple, $-CH_2CH_2-$, $-CH_2O$ ou $-OCH_2-$ ;

$X^1$, $X^2$, $X^3$, $X^4$ sont indépendamment les uns des autres un hydrogène ou un fluor ;

$R^1$ est un hydrogène, un alkyle ou alcényle non substitué ou bien unisubstitué ou plurisubstitué par du fluor ou du chlore possédant 1 ou 2 à 12 atomes de carbone et dans lesquels un ou deux groupes $CH_2$ non contigus peuvent être remplacés par -O-, ou bien un groupe de la formule

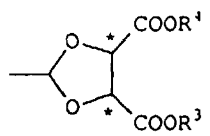

(a)

ou bien, lorsque $R^1$ est lié à un cycle aromatique, également un groupe fluor, chlore, cyano, $-CF_3$, $-OCHF_2$ ou $-OCF_3$ ;

$R^2$, $R^3$, $R^4$, $R^5$ sont des groupes alkyles ou alcényles possédant 1 ou 2 à 8 atomes de carbone ;

n est égal à 0 ou 1 ; et

* marque un centre de chiralité.

**2.** Composés optiquement actifs selon la revendication 1, dans lesquels les centres de chiralité en position 4 et en position 5 du cycle dioxolane présentent tous les deux la configuration [R] ou tous les deux la configuration [S].

**3.** Composés optiquement actifs selon la revendication 1 ou la revendication 2, dans lesquels n = 1.

**4.** Composés optiquement actifs selon l'une quelconque des revendications 1 à 3, dans lesquels $X^1$ et $X^2$ ou $X^3$ et $X^4$ signifient le fluor.

**5.** Composés optiquement actifs selon l'une quelconque des revendications 1 à 4, dans lesquels $R^1$ désigne un groupe alkyle, alcényle, alcoxy, alcényloxy, alcoxyalkyle ou alcoxyalcényle ayant 1 ou 2 à 6 atomes de carbone.

**6.** Composés optiquement actifs des formules :

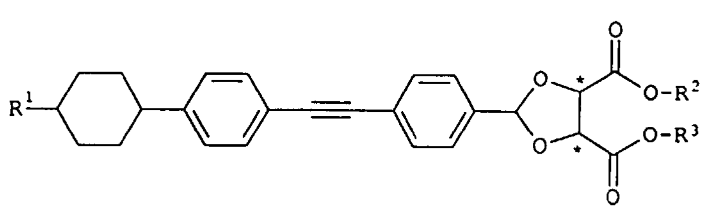

Ia

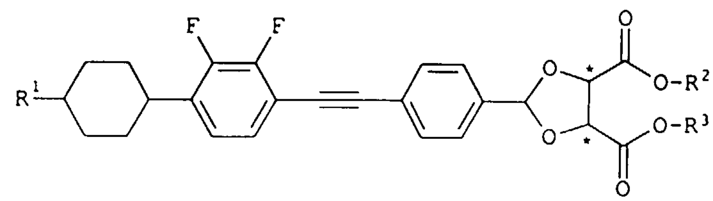

Ib

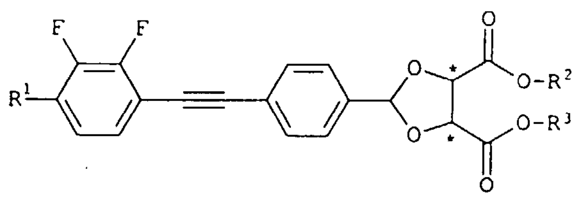

Ic

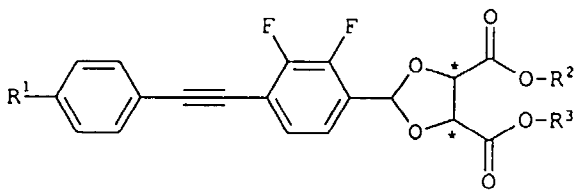

Id

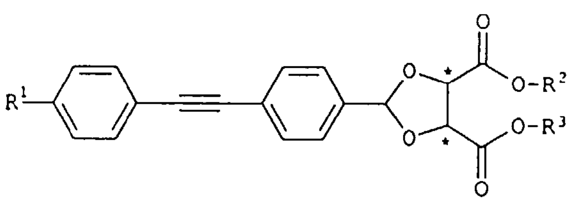

Ie

dans lesquelles $R^1$ est un hydrogène, un groupe alkyle ou alcényle non substitué ou bien unisubstitué ou plurisubstitué par le fluor ou le chlore possédant 1 ou 2 à 12 atomes de carbone et dans lequel un groupe $CH_2$ peut également être remplacé par -O- ou également, dans le cas où $R^1$ est lié à un cycle aromatique, un groupe fluor, chlore, cyano, $-CF_3$, $-OCHF_2$ ou $-OCF_3$ ; et $R^2$ et $R^3$ ont les significations indiquées dans la revendication 1.

**7.** Composés optiquement actifs selon la revendication 1, dans lesquels $R^1$ est un groupe de la formule

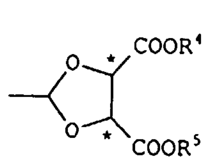

(a)

et n = 0 ou 1.

**8.** Mélange à cristaux liquides contenant au moins un composé selon l'une quelconque des revendications 1 à 7 comme agent dopant chiral.

**9.** Utilisation de composés optiquement actifs de la formule générale I définie dans le revendication 1 pour des dispositifs optiques et électro-optiques.

**10.** Utilisation de mélanges à cristaux liquides selon la revendication 8 pour des dispositifs optiques et électro-optiques.

**11.** Procédé de fabrication de composés de la formule générale

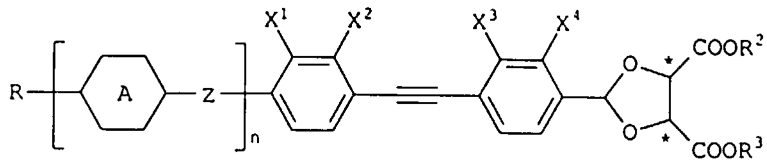

I

dans laquelle $R^1$, $R^2$, $R^3$, n, A, Z, $X^1$, $X^2$, $X^3$ et $X^4$ sont tels que définis dans la revendication 1, caractérisé en ce que l'on couple un dérivé de dioxolane optiquement actif de la formule

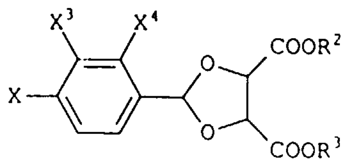

2

dans lequel X désigne un groupe sortant et $X^2$ et $X^3$ ont les significations indiquées dans la revendication 1, à l'aide d'un catalyseur au palladium approprié, avec un dérivé acétylénique de la formule

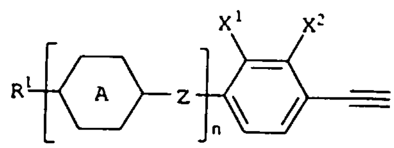

3

dans laquelle $R^1$, A, Z, $X^1$ et $X^2$ sont tels que définis dans la revendication 1.